(19) Europäisches Patentamt / European Patent Office / Office européen des brevets

(11) **EP 1 964 875 B1**

(12) # EUROPEAN PATENT SPECIFICATION

(45) Date of publication and mention
of the grant of the patent:
**07.11.2012 Bulletin 2012/45**

(51) Int Cl.:
*C08J 9/36* (2006.01)        *A61K 8/88* (2006.01)
*A61Q 1/12* (2006.01)

(21) Application number: **06834717.8**

(22) Date of filing: **14.12.2006**

(86) International application number:
**PCT/JP2006/324963**

(87) International publication number:
**WO 2007/069694 (21.06.2007 Gazette 2007/25)**

(54) **POWDER COMPOSED OF INORGANIC COMPOUND-LOADED POLYAMIDE POROUS PARTICLE**

PULVER AUS MIT ANORGANISCHER VERBINDUNG BELADENEM PORÖSEM POLYAMIDTEILCHEN

POUDRE COMPOSEE DE PARTICULES POREUSES DE POLYAMIDE CHARGEES DE COMPOSES INORGANIQUES

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(30) Priority: **14.12.2005 JP 2005360890**

(43) Date of publication of application:
**03.09.2008 Bulletin 2008/36**

(73) Proprietor: **Ube Industries, Ltd.**
**Ube-shi,**
**Yamaguchi-ken 755-8633 (JP)**

(72) Inventors:
• **SHOJI, Tatsuya**
**Ichihara-shi, Chiba, 290-0045 (JP)**
• **KONISHI, Ryo**
**Ichihara-shi, Chiba, 290-0045 (JP)**
• **ASANO, Yukihiko**
**Ichihara-shi, Chiba, 290-0045 (JP)**
• **YAO, Shigeru**
**Ichihara-shi, Chiba, 290-0045 (JP)**

(74) Representative: **Albrecht, Thomas**
**Kraus & Weisert**
**Patent- und Rechtsanwälte**
**Thomas-Wimmer-Ring 15**
**80539 München (DE)**

(56) References cited:
**WO-A1-2004/043411        JP-A- 61 227 520**
**JP-A- 62 215 638           JP-A- 2002 080 629**
**JP-A- 2003 342 409**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

## FIELD OF THE INVENTION

The present invention relates to a powder comprising fine inorganic compound particles-deposited porous polyamide particles in which the fine inorganic compound particles are deposited on the surface as well as in the pores of the polyamide particles. The invention further relates to a cosmetic composition in which the powder comprising inorganic compound particles-deposited porous polyamide particles is dispersed in cosmetic base material.

## BACKGROUND OF THE INVENTION

It has been studied to provide a cosmetic composition such as foundation cream which contains carrier particles of polymer or inorganic material having fine inorganic compound particles deposited on or in the carrier particles. The fine inorganic compound particles are so selected as to show ultraviolet ray-reflection or ultraviolet ray-absorption, whereby the cosmetic composition can show increased shielding to ultraviolet rays.

Further, it has been also studied to incorporate particles of polymer or inorganic material on which an inorganic compound reactive with free fatty acids having been oozed onto a surface of a skin to form metal soaps is deposited into a cosmetic composition such as foundation cream, thereby keeping the coated cosmetic compound for a prolonged period of time.

As for the inorganic compound-deposited particles, the below-described inventions have been already disclosed.

Patent Publication 1 (Japanese Patent Provisional Publication No. 63-27532) discloses inorganic compound-deposited polyamide particles in which fine zirconium oxide particles are deposited on the surface of the polyamide particles. This publication teaches that the zirconium oxide particle-deposited polyamide particles show high reflection to light in the wide range from the ultraviolet region to the near infrared region because the zirconium oxide particle-deposited polyamide particles show light-reflection property similar to the zirconium particles.

Patent Publication 2 (Japanese Patent Provisional Publication No. 9-30935) discloses inorganic compound-deposited polymer or silicon oxide particles which contains fine titanium dioxide particles or fine zinc oxide particles dispersed therein and coated on their surfaces with fine zirconium oxide particles. The thus coated particles are further coated with fine aluminum oxide particles and furthermore subjected to hydrophobic treatment. This publication teaches that the disclosed inorganic compound-deposited particles show good absorption of ultraviolet rays due to the presence of fine particles of titanium dioxide or zinc oxide, good shielding to ultraviolet rays due to the presence of fine zirconium oxide particles having good ultraviolet ray-reflection property, and further show good smoothness and repulsion to water and oil.

Patent Publication 3 (Japanese Patent Provisional Publication No. 61-257909) discloses inorganic compound-deposited particles in which fine particles of zinc oxide and/or zinc carbonate are deposited on the surface of the particles. This publication teaches that a coated cosmetic composition containing the particles on which the fine particles of zinc oxide and/or zinc carbonate are deposited can be kept for an increased period of time because the zinc oxide and zinc carbonate easily react with free fatty acids in sebum having oozed from the inside of skin to form metal soaps.

Patent Publication 4 (Japanese Patent Provisional Publication No. 2002-265624) discloses inorganic compound-deposited porous polymer particles in which fine particles of a compound of metal belonging to I to VIII Groups are deposited. This publication teaches a process for depositing the fine metal compound particles on the porous polymer particles in which the porous polymer particles are dispersed in a solution of a metal compound, whereby the metal compound is absorbed or deposited on the porous polymer particles. According to the description of the publication, the metal compound particles comprise soluble compounds and contain a strong acid or elements derived from the strong acid.

It is desired for a cosmetic composition such as foundation cream to be coated on human skin that the coated cosmetic composition shows high visible light-scattering property or soft focus effects. The high visible light-scattering property inhibits abnormal light scattering (resulting abnormal shining) on the human skin or shields wrinkles and colored spots on the human skin by giving gradation. The studies made by the present inventors have revealed that the inorganic compound is too smoothly coated on the non-porous particles of polymer or inorganic material used for producing the inorganic compound-deposited particles which are disclosed in Patent Publications 1 to 3, so that the coated particles have no rough surfaces and hence satisfactorily high visible light-scattering cannot be expected.

In contrast, the inorganic compound-deposited polymer particles disclosed in Patent Publication 4 use porous polymer particles which have a great number of concaves and convexes on their surfaces. Therefore, it is expected to show high visible-light scattering. However, since a strong acid or an element derived from the strong acid is necessarily contained in the inorganic compound-deposited polymer particles produced in the process described in Patent Publication 4, the produced particles are hardly appropriate as the components to be incorporated in a cosmetic composition to be coated on a human skin.

## SUMMARY OF THE INVENTION

Accordingly, it is an object of the invention to provide a powder comprising inorganic compound-deposited polymer particles which show high visible light-scattering and is not harmful to human skin.

The present invention resides in powder comprising fine inorganic compound particles-deposited porous polyamide particles in which the fine inorganic compound particles are deposited on surfaces and in pores of the porous polyamide particles having pores having a mean pore diameter in the range of 0.01 to 0.5 $\mu$m, the porous polyamide particles have a mean primary particle diameter in the range of 1 to 30 $\mu$m, the fine inorganic compound particles have a mean primary particle diameter in the range of 0.001 to 0.1 $\mu$m, and at least 80% of number of the fine inorganic compound particles contains no strong acidic component, in which the fine inorganic compound particles are contained in an amount of 0.01 to 80 weight % in the powder, in which the porous polyamide particles have a spherulite structure and in which the ratio of the mean primary particle diameter of the fine inorganic compound particles to the mean pore diameter of the porous polyamide particles is in the range of 1/100 to 1/2.

In a preferred embodiment of the powder of the invention which comprises inorganic compound-deposited porous polyamide particles, the inorganic compound particles comprise zinc oxide, aluminum oxide, zirconium oxide or titanium dioxide.

The invention further resides in a cosmetic composition comprising the powder of the invention dispersed in cosmetic material.

The powder of the invention which comprises inorganic compound-deposited porous polyamide particles shows high visible light-scattering and is not harmful to human body because it contains little strong acidic component. Accordingly, the powder of the invention which comprises inorganic compound-deposited porous polyamide particles is favorably employed as component for producing industrial products to be used under such condition that the product is brought into direct contact with the human body, such as cosmetic compositions. The cosmetic compositions containing the powder of the invention are not harmful to human body and favorably inhibit abnormal shining on human skin and favorably shield defective skin conditions such as wrinkles and concaves on human skin.

## BRIEF EXPLANATION OF THE DRAWINGS

Fig. 1 a scanning electron microscopic photograph of the zinc oxide-deposited porous polyamide particle produced in Example 1.

Fig. 2 a transmission electron microscopic photograph of the zinc oxide-deposited porous polyamide particle produced in Example 1.

## PREFERRED EMBODIMENTS OF THE INVENTION

In the invention, the inorganic compound-deposited porous polyamide particles comprise porous polyamide particles having pores on their surfaces and fine inorganic compound particles deposited on the surfaces or in the pores of the polyamide particles. The porous polyamide particles used for the inorganic compound-deposited porous polyamide particles of the invention have a greater number of concaves and convexes on their surfaces, as compared with the non-porous polyamide particles. For this reason, the inorganic compound-deposited porous polyamide particles of the invention show high visible light-scattering.

The porous polyamide particle used in the invention has a spherulite structure. The combination of the spherulite structure and porous structure of the polyamide particle more favorably gives increased light-scattering. In the specification, the polyamide particle having a spherulite structure means that the polyamide particle has such spherulite structure that polymer fibrils grow radially or three isotropic-dimensionally from a single nucleus or plural nuclei produced in the center position. The spherulite structure is formed in a crystalline polymer particle. The spherulite structure of the particle can be determined by observing a section of the particle by means of a transmission electron microscope (TEM) or by observing light transmission of the particle under crossed nicols using a polarization microscope.

The porous polyamide particles on which the inorganic material particles are deposited can be of any form such as spherical, nearly spherical, C letter form having on one side a projecting center portion and one another side a center recess portion, cylinder form, or dumbbell form. The porous polyamide particles comprise preferably at least 70%, more preferably 80%, most preferably 90%, in terms of number of particles, of the porous polyamide particles of the same particle forms. If the porous polyamide particles have various forms, it is not easy to deposit and disperse the fine inorganic compound particles uniformly on the polyamide particles. The porous polyamide particles preferably are in the form of spherical particles.

The porous polyamide particles have a mean primary particle diameter (number average primary particle diameter) in the range of 1 to 30 $\mu$m, preferably 1 to 25 $\mu$m. If the mean primary particle diameter is less than 1 $\mu$m, the polyamide particles are apt to strongly aggregate to form secondary particles. If the mean primary particle diameter is more than 30 $\mu$m, the inorganic compound-deposited porous polyamide particles may give unpleasant feeling when they are incorporated into a cosmetic composition.

A particle size distribution index (PDI) of the porous polyamide is preferably in the range of 1.0 to 2.0, more preferably in the range of 1.0 to 1.5, particularly preferably in the range of 1.0 to 1.3. The particle size distribution index (PDI) is represented by Dn/Dv, in which Dn means a number average primary particle diameter and Dv means a volume average primary particle diameter. The PDI is one of indexes indicating width of particle size distribution of the porous polyamide particles. A PDI near to 1 means the width of particle size distribution is narrow. If the porous polyamide particles are dispersed in a cosmetic composition, it is preferred that the porous polyamide particles show a PDI near to 1. This is because the particles are uniformly dispersed in the cosmetic composition.

The porous polyamide particles have a mean pore size in the range of 0.01 to 0.5 $\mu$m, preferably in the range of 0.01 to 0.3 $\mu$m. If the mean pore size is less than 0.01 $\mu$m, it is difficult to deposit the fine inorganic compound particles on the polyamide particles in an amount enough to impart satisfactorily high visible light-scattering property to the polyamide particles. On the other hand, if the mean pore size is more than 0.5 $\mu$m, the fine inorganic compound particles are deposited in the pores under aggregated conditions. The UV ray-absorbing property of fine inorganic compound particles showing UV ray-absorbing property (e.g., fine titanium dioxide particles and fine zinc oxide particles) decreases when the particles are aggregated.

The porous polyamide particles have a BET specific surface area preferably in the range of 0.1 to 80 $m^2$/g, more preferably in the range of 3 to 75 $m^2$/g, most preferably 5 to 70 $m^2$/g. If the specific surface area is less than 0.1 $m^2$/g, it may be difficult to deposit the fine inorganic compound particles on the polyamide particles in an amount enough to impart satisfactorily high visible light-scattering property to the polyamide particles.

The porous polyamide particles preferably have a porosity index (RI) preferably in the range of 5 to 100, more preferably 10 to 80. The porosity index is represented by $S/S_0$, in which S means a BET specific surface area of the porous polyamide particles and So means a BET specific surface area of non-porous polyamide particles having the same mean particle diameter. If the porosity index (RI) is less than 5, it may be difficult to deposit the fine inorganic compound particles on the polyamide particles in an amount enough to impart satisfactorily high visible light-scattering property to the polyamide particles. The BET specific surface area (So) of non-porous polyamide particles having the same mean particle diameter can be determined from the following formula:

$$S_0 (m^2/kg) = 6/[\rho (kg/m^3) \times Dn (m)],$$

in which $\rho$ means a density of polyamide and Dn is a number average particle diameter.

The porous polyamide particles preferably have a void volume in the range of 30 to 70%. If the void volume is less than 30%, it may be difficult to deposit the fine inorganic compound particles on the polyamide particles in an amount enough to impart satisfactorily high visible light-scattering property to the polyamide particles. If the void volume is more than 70%, it may be difficult for the polyamide particles to keep their forms uniformly and to handle smoothly. The void volume means a ratio of a volume of voids per whole volume of the polyamide particles. The void volume can be determined from the following formula:

$$Void \ volume (\%) = 100 \times P (m^3/kg) / [P (m^3/kg) + 1000/\rho (kg/m^3)],$$

in which P means an accumulated in-particle pore volume of the porous polyamide particles and $\rho$ means a density of porous polyamide particle.

The porous polyamide particles show a boiled linseed oil absorption of preferably not less than 150 mL/100 g (measured according to JIS K 5101), more preferably not less than 200 mL/100 g. If porous polyamide particles having a higher boiled linseed oil absorption are used, the resulting fine inorganic compound-deposited polyamide particles show a higher boiled linseed oil absorption.

The porous polyamide particles can be made of aliphatic, alicyclic, or aromatic polyamide, or copolymer of polyamide. Preferred is aliphatic polyamide. Examples of the aliphatic polyamide include homopolymers such as polyamide 6, polyamide 66, polyamide 11, and polyamide 12 and their copolymers. Preferred aliphatic polyamides are homopolymers such as polyamide 6, polyamide 66 and their copolymers. Most preferred is homopolymer of polyamide 6. The polyamides preferably have more amino groups than carboxyl groups at their terminals.

The polyamide has a molecular weight, preferably in the range of 3,000 to 100,000, more preferably in the range of 5,000 to 40,000, most preferably 6,000 to 20,000.

The porous polyamide particles can be produced by mixing a polyamide solution with a combination of water and non-solvent for the polyamide to temporarily yield a homogeneous mixture solution and subsequently to allow the mixture solution to stand to precipitate the polyamide particles.

The solvent of the polyamide solution can be selected from phenol compounds and formic acid. Examples of the phenol compounds include phenol, o-cresol, m-cresol, p-cresol, cresol acid, and chlorophenol. The crystalline polyamide can be dissolved in these solvents at room temperature or after heating to 30 to 90°C. Most preferred is phenol. Phenol is less harmful as compared with other solvents. The concentration of polyamide in the polyamide solution is preferably in the range of 0.1 to 30 wt.%, more preferably in the range of 0.2 to 25 wt.%.

A freezing-point depressant may be added to the polyamide solution. A non-solvent for polyamide can be employed in an amount not to precipitate the dissolved polyamide. Examples of the freezing-point depressant include water, methanol, ethanol, 1-propanol, 2-propanol, 1-butanol, 2-butanol, 2-methyl-1-propanol, 2-methyl-2-propanol, 1-pentanol, 1-hexanol, ethylene glycol, triethylene glycol, propylene glycol, glycerol, and diglycerol.

The non-solvent for polyamide may be partly compatible (may dissolve a small amount) with a solvent for polyamide (aromatic alcohol or formic acid) or water. Examples of the non-solvents include aliphatic alcohols, aliphatic ketones and their mixtures. Examples of the aliphatic alcohols include methanol, ethanol, 1-propanol, 2-propanol, 1-butanol, 2-butanol, 2-methyl-1-propanol, 2-methyl-2-propanol, 1-pentanol, 1-hexanol, ethylene glycol, triethylene glycol, propylene glycol, and glycerol. Examples of the aliphatic ketones include acetone and methyl ethyl ketone.

In the production of the porous polyamide particles, the polyamide solution, non-solvent and water can be mixed in optionally selected manner , so far as the mixture of the polyamide solution, non-solvent and water temporarily forms a homogeneous solution. For instance, 1) to the polyamide solution are sequentially added the non-solvent and water; 2)the non-solvent and water are first mixed, and to the mixture is added the polyamide solution; or 3) water is first added to the polyamide solution, and then the non-solvent is added. In the above-mentioned procedure 2), the polyamide solution can be added at once or in two or more portions.

In the production of the porous polyamide particles, the mixture solution can be stirred to temporarily yield a homogenous solution. Subsequently, the mixture is allowed to stand preferably at 0 to 80°C, more preferably 20 to 40°C, to precipitate the porous polyamide particles.

The precipitated polyamide particles can be isolated from the solution by one of the conventional procedures such as decantation, centrifugal separation and filtration. For instance, an aliphatic alcohol such as methanol, ethanol or propanol, water or acetone is added to the solution in which the polyamide particles are precipitated, and subsequently the polyamide particles can be isolated by decantation or centrifugal separation. The polyamide particles can be further washed with several portions of an aliphatic alcohol such as methanol, ethanol or propanol, water or acetone and then isolated by decantation or centrifugal separation. Further, the polyamide particles can be subjected to hot air drying, spray drying, stirring drying or vacuum drying.

The porous polyimide particles obtained above can be brought into contact with a non-solvent (which is compatible with the solvent of the polyamide solution at a temperature of 40°C or higher), thereby extracting the solvent out of the porous polyamide particles. Examples of the non-solvent employable for extracting the solvent include an aliphatic alcohol, an aliphatic or aromatic ketone, an aliphatic or aromatic hydrocarbon, and water. The non-solvent can be used alone or in combination. The non-solvent preferably cannot dissolve more than 0.01 wt.% of polyamide at 40°C. The aliphatic alcohol can be a monohydric aliphatic alcohol having 1 to 3 carbon atoms such as methanol, ethanol, 1-propanol, or 2-propanol. The aliphatic ketone can be acetone or methyl ethyl ketone. The aromatic ketone can be acetophenone, propiophenone, or butyrophenone. The aromatic hydrocarbon can be toluene or xylene. The aliphatic hydrocarbon can be heptane, hexane, oxtane or n-decane.

At least 80% in terms of number, preferably at least 90%, more preferably 100%, of the fine inorganic compound particles to be deposited on the porous polyimide particles do not contain a strong acid component.

The fine inorganic compound particles have a mean primary particle diameter in the range of 0.001 to 0.1 $\mu$m. The ratio of the mean primary particle diameter of the fine inorganic compound particles to the mean pore diameter of the porous polyamide particles is preferably in the range of 1/100 to 1/2, more preferably in the range of 1/50 to 1/5. The fine inorganic compound particles may be in any form such as sphere, lump, mass, needle, or rod.

Examples of the fine inorganic compound particles include fine particles of oxides, nitrides and carbides. Examples of the fine inorganic compound particles containing no strong acid component include iron oxide (iron oxide yellow, iron oxide red, or iron oxide black), titanium dioxide, zinc oxide, aluminum oxide, silicon oxide, zirconium oxide, cerium oxide, silicon carbide, organic pigments, ultramarine pigment, blue pigment, carbon black, lead oxide, germanium oxide, indium oxide, tin oxide, antimony-doped tin oxide, indium-tin complex oxide, silica-lithium complex oxide, magnetite, maghemite, manganese-zinc ferrite, rare earth metal-iron garnet, nickel-zinc ferrite, titanium nitride, zirconium nitride, silicon nitride, boron carbide, boron nitride, hydroxyapatite, $\alpha$-calcium phosphate, $\beta$-calcium phosphate, $\gamma$-calcium phosphate, octacalcium phosphate, clay such as mont-morillonite, mica, talc, or pigment of their complex material.

Preferred are fine particles of zinc oxide, aluminum oxide, zirconium oxide or titanium dioxide. Most preferred are fine particles of zinc oxide. The zinc oxide particles can react with a free fatty acid in the sebum to form a metal soap as is described hereinbefore. The porous polyamide particles show excellent oil absorption. Therefore, if the fine zinc oxide particle-deposited porous polyamide particles are incorporated into a cosmetic composition, the sebum-removing characteristic of the cosmetic composition is improved. Accordingly, the cosmetic composition containing the fine zinc oxide particle-deposited porous polyamide particles coated on the skin is kept on the skin for a long period of time. The fine inorganic compound particles can be employed in combination.

The fine inorganic compound particles can be deposited on the porous polyamide particles by the steps of preparing a slurry by dispersing the fine inorganic compound particles and porous polyamide particles in a dispersing medium, stirring the slurry, and drying the slurry.

The dispersing medium preferably is water, a water-compatible organic solvent, or a mixture thereof. Examples of the water-compatible organic solvent include acetone, methanol, ethanol, 1-propanol, 2-propanol, 1-butanol, 2-butanol, 1-pentanol, 1-hexanol, ethylene glycol, triethylene glycol, propylene glycol, glycerol, diglycerol, and their mixtures.

The slurry can be prepared by mixing a slurry containing the fine inorganic compound particles and a slurry containing the porous polyamide particles; adding the porous polyamide particles to a slurry containing the fine inorganic compound particles; or adding the fine inorganic compound particles to a slurry containing the porous polyamide particles.

The slurry can be stirred by means of a combination of a stirring paddle and a three-one motor, a magnetic stirrer, a ultrasonic homogenizer, and a combination of these stirring means.

The stirred slurry containing the porous polyamide particles on which the fine inorganic compound particles are deposited is then subjected to filtration or centrifugal separation to recover the fine inorganic compound particles-deposited porous polyamide particles. The recovered deposited porous polyamide particles are then dried by vacuum drying or a dryer in which humidity is kept constant. The slurry containing the fine inorganic compound particles-deposited porous polyamide particles can be directly subjected to spray drying to obtain dry fine inorganic compound particles-deposited porous polyamide particles.

Alternatively, the fine inorganic compound particles can be deposited on the porous polyamide particles by subjecting the fine inorganic compound particles and the porous polyamide particles to dry blending. The dry blending can be carried out by any procedures, provided that the porous polyamide particles are not damaged, for instance, the porous structure on the surface is broken or diminished or the polyamide particles are not melted, by the high machine blending energy or energy produced by collision of the particles. For instance, a rocking mixer (available from Aichi Electric Co., Ltd.) or IKA-VIBRAX VXR basic (small size shaker, available by Ika Japan Co., Ltd.) can be employed for rotating or mixing the particles.

A surface active agent can be applied to either or both of the fine inorganic compound particles and porous polyamide particles in advance of depositing the former fine particles on the latter particles, whereby firmly fixing the former fine particles onto the latter particles. The surface active agent can be anionic, cationic or nonionic.

The anionic surface active agent can be a fatty acid soap such as sodium laurate or sodium palmitate, higher alkyl nitrate ester salt, alkylether sulfate ester salt, higher fatty acid amide sulfonate salt, or phosphate ester salt. The cationic surface active agent can be alkyltrimethyl ammonium chloride or dialkyldimethylammonium chloride. The nonionic surface active agent can be (poly)-glycerol fatty acid ester, sorbitan fatty acid ester, sugar fatty acid ester, polyoxyalkylene (2 or 3 carbon atoms) alkyleter, polyoxyethylene fatty acid ester, polyoxyethylene sorbitan fatty acid ester, polyoxyethylene hardened castor oil, polyoxyethylene hardened caster oil fatty acid ester, or polyoxyethylene-polyoxypropylene block copolymer.

The inorganic compound-deposited porous polyamide particles of the invention are favorably employable as components of the cosmetic composition. However, they also can be utilized as functional particles for producing optical elements of electronic devices, paints, clinical tools, or foods.

The cosmetic composition of the invention comprises the inorganic compound-deposited porous polyamide particles of the invention in the cosmetic base material. The cosmetic base material is medium manufacturing a cosmetic composition. The cosmetic base material can be oily material, aqueous material, powdery material, film-forming polymer material used for pack, surface active agent serving as emulsifier, or their mixture.

The oily material can be oil, fatty, wax, hydrocarbon, and higher fatty acid. The aqueous material can be purified water or a lower alcohol such as ethanol. The powder material can be an inorganic pigment such as talc or kaolin. The polymer material can be natural or synthetic polymer compound. The surface active agent can be a nonionic surface active agent or an anionic surface active agent.

The cosmetic base material can further contain additives such as detergent, humectant, softener, astringent, UV shielding agent, colorant, perfume, deodorant, thickener, antiseptic, pH adjusting agent, metal chelating agent, cell activator, blood flow accelerating agent, whitener, sebum restrainer, bactericide, anti-inflammatory agent, and sweat restrainer.

EXAMPLES

In the following examples, the mean primary particle diameter (number average primary particle diameter), volume average primary particle diameter, particle size distribution index (PDI), BET specific surface area, mean pore diameter, porosity index (RI), void volume, crystallinity, boiled linseed oil absorption, luminous reflectance, and zinc oxide content were determined by the below-described manner.

[Number average primary particle diameter, volume average primary particle diameter, Particle size distribution index (PDI)]

The particle sizes of 100 particles are observed by means of a scanning electron microscope, and the desired diameters and index are calculated in the known manner.

[BET Specific surface area]

The known BET three points measuring method using nitrogen adsorption is employed.

[Mean pore diameter]

A mercury porosimeter is employed for measuring the pore diameter in the range of 0.0036 to 14 $\mu$m.

[Porosity index (RI)]

The above-obtained BET specific surface area is applied to the aforementioned formula. The density of polyamide 6 is 1,180 kg/m$^3$.

[Void volume]

The accumulated pore volume is measured by means of a mercury porosimeter, and a graph of the accumulated pore volume against the pore diameter is illustrated. The accumulated pore volume from a pore diameter at the largest inflection point to a pore diameter at a point less than the largest inflection point by 0.035 $\mu$m is considered as the "in-particle accumulated pore volume". The void volume is then calculated according to the aforementioned formula. The density of polyamide 6 is 1,180 kg/m$^3$.

[Crystallinity]

The amount of heat of fusion of polyimide particles is measured by a differential scanning calorimeter (DSC) in a nitrogen stream at a flow rate of 40 mL/min., under the condition that the temperature elevation rate is set to 5°C/min. The amount of heat of fusion is placed in the aforementioned formula to give the crystallinity. The polyamide 6 having a crystallinity of 100% has an amount of heat of fusion of 189 J/g.

[Boiled linseed oil absorption]

The measurement is made by the method defined in JIS K 5101.

[Luminous reflectance]

0.2 g of the powder is placed uniformly on one surface of a double side adhesive coated transparent tape (10 cm x 10 cm) to give a specimen. The specimen is placed in an angle-variable spectroscopic colorimeter Color Robot III (available from Color Techno System Co., Ltd.) to measure a luminar reflectance at reflection angles of 0°, 20° and 45° detected upon receipt of a fixed angle of incidence at 45°. A ratio of the quantity of the reflected light to the quantity of the incident light at each angle in terms of percent (namely, luminous reflectance) is calculated.

[Amount of zinc oxide]

5 mg of the powder is decomposed in a sulfuric acid-nitric acid mixture, and the amount of zinc is measured by ICP emission analysis. The amount of zinc is then converted to an amount of zinc oxide.

[Example 1]

100 g of polyamide 6 (number average molecular weight: 8,000, 1010X1 available from Ube Industries, Ltd.) was added to 810 g of phenol heated to 70°C, and the resulting mixture was stirred until the polyamide 6 is completely dissolved. Further, 90 g of methanol was added, and the mixture was then stirred under cooling, to give 1 kg of a polyamide 6 solution (polyamide concentration: 10 wt.%). To the polyamide 6 solution (1 kg) adjusted to 20°C were added 7 kg of methanol and 0.5 kg of water. The resulting mixture was stirred until a homogeneous mixture solution was formed, and the homogeneous mixture solution was allowed to stand. After confirming that polyamide 6 particles precipitated in the mixture solution, the mixture solution was further allowed to stand for 2 hours. Subsequently, the polyamide 6 particles were collected on a filter and washed on the filter with 5 portions of methanol (10,000 mL) at 25°C. The thus washed polyamide 6 particles were dried first at 60°C for 8 hours using an air dryer and then dried at 60°C for 8 hours in a vacuum dryer. The dried polyamide 6 particles (10 g) were placed in a Soxhlet extractor equipped with heating means, and then extraction was carried out by refluxing methanol for 10 hours, thereby bringing methanol into contact with the polyamide 6 particles. The polyamide 6 particles were taken out of the Soxhlet extractor and dispersed in methanol to prepare a 10 wt.% slurry. The slurry was spray dried at 180°C by means of a spray dryer, to obtain a polyamide 6 powder.

It was confirmed by means of a scanning electron microscope that the resulting polyamide 6 powder was composed of substantially uniform spherical porous particles. It was further confirmed by observation of section of the resulting polyamide 6 powder using transmission electron microscope (TEM) that crystal was grown from the center nucleus in each particle. This means that each particle had a spherulite structure. The polyamide 6 particle was then observed by means of polarization microscope. It was confirmed that the polyamide 6 particle transmitted light under crossed nicols. This result confirms that the polyamide 6 particle had a spherulite structure.

The mean primary particle diameter (number average primary particle diameter), volume average primary particle diameter, particle size distribution index (PDI), BET specific surface area, mean pore diameter, porosity index (RI), void volume, crystallinity, boiled linseed oil absorption, and luminous reflectance were determined on the porous polyamide 6 particles. The following data were obtained:

| | |
|---|---|
| mean primary particle diameter: | 8.2 $\mu$m |
| volume average primary particle diameter: | 11.8 $\mu$m |
| PDI: | 1.43 |
| BET specific surface area: | 28.2 m$^2$/g |
| mean pore diameter: | 0.095 $\mu$m |
| porosity index (RI): | 45.0 |
| void volume: | 65% |
| crystallinity: | 57% |
| boiled linseed oil absorption: | 210 mL/100 g |
| luminous reflectance: | 26.44% (reflection angle 0°) |
| | 30.62% (reflection angle 20°) |
| | 68.02% (reflection angle 45°). |

In a glass sample bottle were placed 1 g of the porous polyamide 6 powder and 0.5 g of zinc oxide powder (mean primary particle diameter: 0.010 $\mu$m, luminous reflectance: 4.39% (reflection angle 0°), 10.07% (reflection angle 20°), more than 160.0% (reflection angle 45°), FINEX-75, available from Sakai Chemical Industries, Co., Ltd.). The sample bottle was then mechanically stirred at 2,000 rpm in a small size shaker (IKA-VIBRAX VXR BASIC, available from Ika Japan, Co., Ltd.) for 2 hours, whereby the porous polyamide 6 powder and the zinc oxide powder were mixed. The resulting mixture was placed on a filter (pore size: 3 $\mu$m), washed with methanol, and finally dried in a vacuum dryer (60°C) for 8 hours.

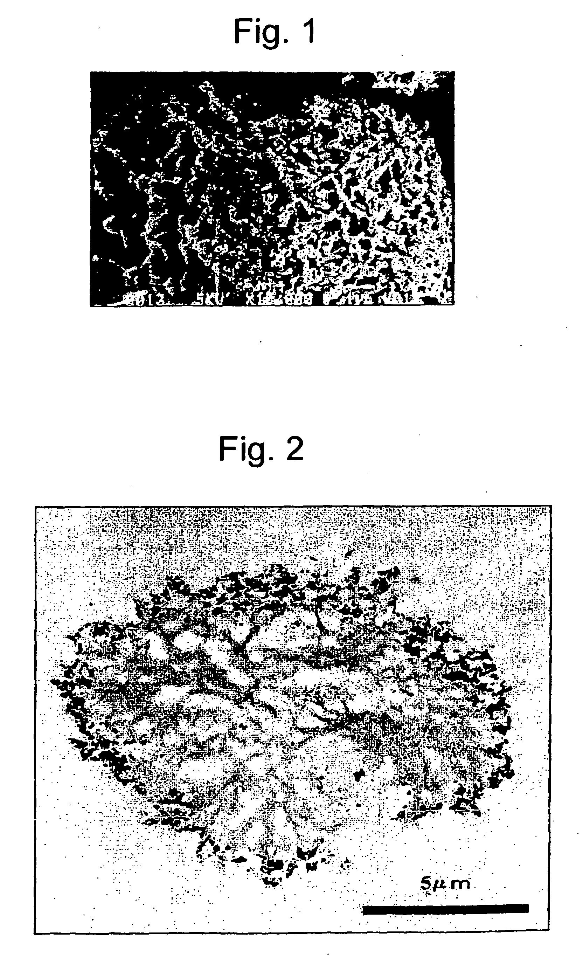

The resulting powder mixture was observed by means of a scanning electron microscope and a transmission electron microscope. The scanning electron microscopic photograph is shown in Fig. 1, and the transmission electron microscopic photograph is shown in Fig. 2.

From Fig. 1 and Fig. 2, it was confirmed that the powdery mixture was composed of porous polyamide 6 particles on which many fine zinc oxide particles were deposited. The fine zinc oxide particles were present on the surface and in the pores of the porous polyamide 6 particles.

The following data were obtained on the fine zinc oxide particles-deposited porous polyamide 6 particles:

| | |
|---|---|
| zinc oxide content: | 17.4 wt.% |

(continued)

| BET specific surface area: | 26.5 m$^2$/g |
| mean pore diameter: | 0.082 μm |
| luminous reflectance: | 35.20% (reflection angle 0°) |
| | 37.79% (reflection angle 20°) |
| | 40.53% (reflection angle 45°). |

[Example 2]

The procedures of Example 1 were repeated except that 0.3 g of the fine zinc oxide powder was used, to produce a powdery mixture.

The observations using a scanning electron microscope and a transmission electron microscope indicated that the powdery mixture was composed of porous polyamide 6 particles on which many fine zinc oxide particles were deposited. The fine zinc oxide particles were present on the surface and in the pores of the porous polyamide 6 particles.

The following data were obtained on the fine zinc oxide particles-deposited porous polyamide 6 particles:

| zinc oxide content: | 11.7 wt.% |
| BET specific surface area: | 25.5 m$^2$/g |
| mean pore diameter: | 0.073 μm |
| luminous reflectance: | 33.68% (reflection angle 0°) |
| | 36.04 (reflection angle 20°) |
| | 42.84% (reflection angle 45°). |

[Example 3]

The procedures of Example 1 were repeated except that 0.1 g of the fine zinc oxide powder was used, to produce a powdery mixture.

The observations using a scanning electron microscope and a transmission electron microscope indicated that the powdery mixture was composed of porous polyamide 6 particles on which many fine zinc oxide particles were deposited. The fine zinc oxide particles were present on the surface and in the pores of the porous polyamide 6 particles.

The following data were obtained on the fine zinc oxide particles-deposited porous polyamide 6 particles:

| zinc oxide content: | 5.6 wt.% |
| BET specific surface area: | 24.2 m$^2$/g |
| mean pore diameter: | 0.073 μm |
| luminous reflectance: | 33.42% (reflection angle 0°) |
| | 36.00 (reflection angle 20°) |
| | 44.08% (reflection angle 45°). |

The luminous reflectance of the powder mixture produced in each of Examples 1 to 3 and the luminous reflectance of each of the porous polyamide 6 particles and fine zinc oxide particles used for the production of the powder mixture are set forth in Table 1.

Table 1

| Content of zinc oxide | | luminous reflectance | | |
|---|---|---|---|---|
| | | 0° | 20° | 45° |
| Example 1 | 17.4 wt.% | 35.20 | 37.79 | 40.53 |
| Example 2 | 11.7 wt.% | 33.68 | 36.04 | 42.84 |
| Example 3 | 5.6 wt.% | 33.42 | 36.00 | 44.08 |
| Porous polyamide 6 particles | | 26.44 | 30.62 | 68.02 |
| Fine zinc oxide particles | | 4.39 | 10.07 | ≥160.0 |

From the results set forth in Table 1, it is understood that the powdery mixtures show higher visible ray scattering as compared with the porous polyamide 6 particles and fine zinc oxide particles, because the luminous reflectance values do not largely vary within a wide range of the reflection angle.

[Example 4]

In a 2 L-volume separable flask equipped with a stirrer having three paddles were placed 750 g of isopropanol and 450 g of water. The temperature of the resulting mixture was adjusted to 25°C, and the stirrer was rotated at 500 rpm to prepare a mixture of isopropanol and water. Separately, polyamide 6 (number average molecular weight: 13,000) was dissolved in a mixture of phenol and isopropanol (90:10 by weight), to give 30 g of a polyamide solution (concentration: 10 wt.%). The polyamide solution (30 g) was added to the mixture of isopropanol and water under stirring -- First addition. At a lapse of 30 minutes after termination of the first addition, 120 g of the same polyamide solution as the polyamide solution used in the first addition was added to the solution obtained in the first addition -- Second addition. Thus, a polyamide-containing mixture solution was produced.

After confirming that polyamide 6 particles precipitated in the mixture solution, the mixture solution was further allowed to stand for one hour. The polyamide 6 particles were recovered from the mixture solution by suction filtration, repeatedly washed with isopropanol, and dried to obtain a polyamide 6 powder.

It was confirmed by means of a scanning electron microscope that the resulting polyamide 6 powder was composed of substantially uniform spherical porous particles. It was further confirmed by observation of section of the resulting polyamide 6 powder using transmission electron microscope (TEM) that crystal was grown from the center nucleus in each particle. This means that each particle had a spherulite structure. The polyamide 6 particle was then observed by means of polarization microscope. It was confirmed that the polyamide 6 particle transmitted light under crossed nicols. This result confirms that the polyamide 6 particle had a spherulite structure.

The mean primary particle diameter (number average primary particle diameter), volume average primary particle diameter, particle size distribution index (PDI), BET specific surface area, mean pore diameter, porosity index (RI), void volume, crystallinity, and boiled linseed oil absorption were determined on the porous polyamide 6 particles. The following data were obtained:

| | |
|---|---|
| mean primary particle diameter: | 11.1 $\mu$m |
| volume average primary particle diameter: | 12.3 $\mu$m |
| PDI: | 1.11 |
| BET specific surface area: | 19.8 m$^2$/g |
| mean pore diameter: | 0.102 $\mu$m |
| porosity index (RI): | 43.2 |
| void volume: | 65% |
| crystallinity: | 57% |
| boiled linseed oil absorption: | 195 mL/100 g |

In the manner described in Example 1, 1 g of the porous polyamide 6 powder and 0.5 g of zinc oxide powder were mixed to give a powdery mixture.

The observations using a scanning electron microscope and a transmission electron microscope indicated that the powdery mixture was composed of porous polyamide 6 particles on which many fine zinc oxide particles were deposited. The fine zinc oxide particles were present on the surface and in the pores of the porous polyamide 6 particles.

The following data were obtained on the fine zinc oxide particles-deposited porous polyamide 6 particles:

| | |
|---|---|
| zinc oxide content: | 16.7 wt.% |
| BET specific surface area: | 18.2 m$^2$/g |
| mean pore diameter: | 0.092 $\mu$m |
| luminous reflectance: | 35.14% (reflection angle 0°) |
| | 37.20 (reflection angle 20°) |
| | 40.79% (reflection angle 45°). |

[Example 5]

1 g of the porous polyamide 6 powder obtained in Example 1 and 0.5 g of a fine zinc oxide powder (mean primary particle diameter: 0.010 μm) were placed in a glass sample bottle, and the porous polyamide 6 powder and fine zinc oxide powder were mixed using a small size shaker in the manner described in Example 1. The resulting powder mixture (1.5 g) was placed in a 20 mL-volume sample bottle. Subsequently, 2.2 g of oleic acid and a stirrer chip (diameter: 15 mm) were placed in the sample bottle. The stirrer chip was rotated at 100 rpm to mix the powdery mixture and oleic acid. It was observed that the oleic acid reacted with zinc oxide to form solid metal soap. After approx. 2 minutes from the start of the rotation, the rotation of the stirrer chip was clogged. Thus, it was confirmed that the zinc oxide-deposited porous polyamide particles would easily react with oleic acid to form a metal soap.

[Comparison Example 1]

1 g of a commercially available non-porous polyamide 6 powder and 0.5 g of a fine zinc oxide powder (mean primary particle diameter: 0.010 μm) were placed in a glass sample bottle, and the non-porous polyamide 6 powder and fine zinc oxide powder were mixed using a small size shaker in the manner described in Example 1. The resulting powder mixture (1.5 g) was placed in a 20 mL-volume sample bottle. Subsequently, 2.2 g of oleic acid and the stirrer chip were placed in the sample bottle. The stirrer chip was rotated at 100 rpm to mix the powdery mixture and oleic acid. After approx. 13 minutes 30 seconds, it was observed that the oleic acid reacted with zinc oxide to form solid metal soap. After approx. 2 minutes from the start of the rotation, the rotation of the stirrer chip was clogged.

[Example 6]

To 6 weight parts of the zinc oxide-deposited porous polyamide particles obtained in Example 1 were added the below-mentioned materials A to F, and water was added to give 100 weight parts of a mixture. The mixture was uniformly mixed to give foundation cream. The obtained foundation cream was coated on skin. When light was applied to the coated skin, no light glittering was observed.

Material A: mixture of cyclomethicone (22 weight parts) and cetylmethicone (0.2 weight part)
Material B: mixture of mica (0.1 weight part), silica (1 weight part), titanium (7.5 weight parts) and zinc oxide (2.0 weight parts)
Material C: mixture of iron oxide black pigment (0.17 weight part), iron oxide red pigment (0.52 weight part) and iron oxide yellow pigment (1.82 weight parts)
Material D: mixture of trihydroxystearin (0.3 weight part) and cyclomethicone (1.0 weight part)
Material E: propyl p-hydroxybenzoate (0.75 weight part)
Material F: mixture of glycerol (8.0 weight parts), polyvinylpyrrolidone (0.5 weight part), sodium chloride (2.0 weight parts), sodium dehydroacetate (0.3 weight part), phenoxyethanol (0.25 weight part), and EDTA tetrasodium salt (1.0 weight part)

**Claims**

1. Powder comprising fine inorganic compound particles-deposited porous polyamide particles in which the fine inorganic compound particles are deposited on surfaces and in pores of the porous polyamide particles having pores having a mean pore diameter in the range of 0.01 to 0.5 μm, the porous polyamide particles have a mean primary particle diameter in the range of 1 to 30 μm, the fine inorganic compound particles have a mean primary particle diameter in the range of 0.001 to 0.1 μm, and at least 80% of number of the fine inorganic compound particles contains no strong acidic component, in which the fine inorganic compound particles are contained in an amount of 0.01 to 80 weight % in the powder, in which the porous polyamide particles have a spherulite structure and in which the ratio of the mean primary particle diameter of the fine inorganic compound particles to the mean pore diameter of the porous polyamide particles is in the range of 1/100 to 1/2.

2. The powder of claim 1, in which the fine inorganic compound particles comprise zinc oxide, aluminum oxide, zirconium oxide or titanium dioxide.

3. A cosmetic composition comprising the powder of claim 1 dispersed in cosmetic material.

**Patentansprüche**

1. Pulver, das poröse Polyamidpartikel mit darauf abgeschiedenen feinen anorganischen Verbindungspartikeln umfasst, in dem die feinen anorganischen Verbindungspartikel an Oberflächen und in Poren der porösen Polyamidpartikel, die Poren mit einem durchschnittlichen Porendurchmesser im Bereich von 0,01 bis 0,5 μm haben, abgeschieden sind, wobei die porösen Polyamidpartikel einen durchschnittlichen Primärpartikeldurchmesser im Bereich von 1 bis 30 μm haben, die feinen anorganischen Verbindungspartikel einen durchschnittlichen Primärpartikeldurchmesser im Bereich von 0,001 bis 0,1 μm haben und wenigstens 80% der Zahl der feinen anorganischen Verbindungspartikel keine stark saure Komponente enthalten, wobei die feinen anorganischen Verbindungspartikel in einer Menge von 0,01 bis 80 Gew.-% im Pulver enthalten sind, wobei die porösen Polyamidpartikel eine Sphärulitstruktur haben und wobei das Verhältnis des durchschnittlichen Primärpartikeldurchmessers der feinen anorganischen Verbindungspartikel zu dem durchschnittlichen Porendurchmesser der porösen Polyamidpartikel im Bereich von 1/100 bis 1/2 liegt.

2. Pulver gemäß Anspruch 1, wobei die feinen anorganischen Verbindungspartikel Zinkoxid, Aluminiumoxid, Zirkoniumoxid oder Titandioxid umfassen.

3. Kosmetische Zusammensetzung, die das Pulver gemäß Anspruch 1 in kosmetischem Material dispergiert umfasst.


**Revendications**

1. Une poudre comprenant des particules fines de composé inorganique déposées aux particules poreuses de polyamide, dans laquelle les particules fines de composé inorganique sont déposées sur des surfaces et dans des pores des particules poreuses de polyamide qui ont des pores ayant un diamètre moyen des pores dans la plage de 0,01 à 0,5 μm; les particules poreuses de polyamide ont un diamètre moyen des particules primaires dans la plage de 1 à 30 μm; les particules fines de composé inorganique ont un diamètre moyen des particules primaires dans la plage de 0,001 à 0,1 μm et au moins 80% du nombre des particules fines de composé inorganique ne contiennent pas de composant fortement acide, dans laquelle les particules fines de composé inorganique sont contenues dans une quantité de 0,01 à 80% en poids de la poudre, dans laquelle les particules poreuses de polyamide ont une structure sphérolitique et dans laquelle le rapport du diamètre moyen des particules primaires des particules fines de composé inorganique au diamètre moyen des pores des particules poreuses de polyamide est compris dans la plage de 1/100 à 1/2.

2. La poudre selon la revendication 1, dans laquelle les particules fines de composé inorganique comprennent de l'oxyde de zinc, l'oxyde d'aluminium, l'oxyde de zirconium ou le dioxyde de titane.

3. Composition cosmétique comprenant la poudre selon la revendication 1 dispersée dans un matériau cosmétique.

# Fig. 1

# Fig. 2

**EP 1 964 875 B1**

**REFERENCES CITED IN THE DESCRIPTION**

**Patent documents cited in the description**

- JP 63027532 A
- JP 9030935 A
- JP 61257909 A
- JP 2002265624 A